# EUROPEAN PATENT SPECIFICATION

(11) **EP 2 120 976 B1**
(45) Date of publication and mention of the grant of the patent: **15.08.2018**
(21) Application number: 08737391.6
(22) Date of filing: 28.02.2008
(51) Int. Cl.: A61K 35/34, A61P 43/00

(54) **METHODS FOR THE TREATMENT OF ANAL INCONTINENCE USING MYOBLASTS**
VERFAHREN ZUR BEHANDLUNG ANALER INKONTINENZ MIT VERWENDUNG VON MYOBLASTEN
PROCÉDÉS DE TRAITEMENT DE L'INCONTINENCE ANALE EN UTILISANT DES MYOBLASTES

(30) Priority: 28.02.2007 US 892095 P; 27.02.2008 US 38410
(43) Date of publication of application: 25.11.2009
(73) Proprietor: Innovacell Biotechnologie AG, 6020 Innsbruck (AT)
(72) Inventor: MARKSTEINER, Rainer, A-6130 Schwaz (AT)
(74) Representative: Dehmel, Albrecht
(86) International application number: PCT/IB2008/000829
(87) International publication number: WO 2008/104883

(56) References cited:
- WO-A-96/40304
- WO-A1-2004/055174
- WO-A1-2008/076435
- WO-A2-01/32129
- WO-A2-2008/031957
- US-A1- 2003 077 244
- US-A1- 2006 257 445
- US-A1- 2007 224 173
- YIOU R ET AL: "Muscle Precursor Cell Autografting in a Murine Model of Urethral Sphincter Injury" BJU INTERNATIONAL, BLACKWELL SCIENCE, OXFORD, GB, vol. 89, 1 January 2002 (2002-01-01), pages 298-302, XP003009147 ISSN: 1464-4096
- CANNON T W ET AL: "Improved Sphincter Contractility After Allogenic Muscle-derived Progenitor Cell Injection into the Denervated Rat Urethra" UROLOGY, BELLE MEAD, NJ, US, vol. 62, no. 5, 1 January 2003 (2003-01-01), pages 958-963, XP003009146 ISSN: 0090-4295
- KWON D ET AL: "Periurethral cellular injection: Comparison of muscle-derived progenitor cells and fibroblasts with regard to efficacy and tissue contractility in an animal model of stress urinary incontinence" UROLOGY 200608 US, vol. 68, no. 2, August 2006 (2006-08), pages 449-454, XP002485714 ISSN: 0090-4295 1527-9995
- MALOUF A J ET AL: "Internal anal sphincter augmentation for fecal incontinence using injectable silicone biomaterial" DISEASES OF THE COLON AND RECTUM 2001 US, vol. 44, no. 4, 2001, pages 595-600, XP002485715 ISSN: 0012-3706 cited in the application
- VAIZEY C J ET AL: "Injectable bulking agents for treating faecal incontinence" BRITISH JOURNAL OF SURGERY 200505 GB, vol. 92, no. 5, May 2005 (2005-05), pages 521-527, XP002485716 ISSN: 0007-1323 cited in the application
- LORENZI B ET AL: "Treatment of experimental injury of anal sphincters with primary surgical repair and injection of bone marrow-derived mesenchymal stem cells" DISEASES OF THE COLON AND RECTUM 200804 US, vol. 51, no. 4, April 2008 (2008-04), pages 411-420, XP002485717 ISSN: 0012-3706 1530-0358
- KANG S-B ET AL.,: "Sphincter Contractility After Muscle-Derived Stem Cells Autograft into the Cryoinjured Anal Sphincters of Rats" DISEASES OF THE COLON AND RECTUM 2008 US, vol. 0, 2008, pages 1-7, XP002485718
- FEKI A ET AL: "Sphincter incontinence: Is regenerative medicine the best alternative to restore urinary or anal sphincter function?" INTERNATIONAL JOURNAL OF BIOCHEMISTRY AND CELL BIOLOGY 2007 GB, vol. 39, no. 4, April 2007 (2007-04), pages 678-684, XP002485719 ISSN: 1357-2725
- P. Conort ET AL: "Biomatériaux synthétiques injectables : agents de comblement pour le traitement de l'incontinence et du reflux vésico-urétéral", Progrès en Urologie, vol. 15, 1 January 2005 (2005-01-01), pages 942-952, XP055209096, Retrieved from the Internet: URL:http://urofrance.org/fileadmin/documen ts/data/PU/2005/PU-2005-00150942-5/TEXF-PU -2005-00150942-5.PDF [retrieved on 2015-08-24]
- Daniel Skuk ET AL: "Progress in myoblast transplantation: a potential treatment of dystrophies", Microscopy Research and Technique, 15 February 2000 (2000-02-15), pages 213-222, XP055209100, New York DOI: 10.1002/(SICI)1097-0029(20000201/15)48:3/4 <213::AID-JEMT9>3.0.CO;2-Z Retrieved from the Internet: URL:http://onlinelibrary.wiley.com/doi/10. 1002/(SICI)1097-0029(20000201/15)48:3/4<21 3::AID-JEMT9>3.0.CO;2-Z/abstract
- DANIEL SKUK ET AL: "Myoblast transplantation: the current status of a potential therapeutic tool for myopathies", JOURNAL OF MUSCLE RESEARCH AND CELL MOTILITY, KLUWER ACADEMIC PUBLISHERS, DO, vol. 24, no. 4-6, 1 August 2003 (2003-08-01), pages 285-300, XP019256155, ISSN: 1573-2657
- HAKELIUS LARS ET AL: "A new treatment of anal incontinence in children: Free autogenous muscle transplantation", JOURNAL OF PEDIATRIC SURGERY, vol. 13, no. 1, February 1978 (1978-02), pages 77-82, XP028842679, ISSN: 0022-3468, DOI: 10.1016/S0022-3468(78)80216-4
- "Neuromuscular Anatomy of Esophagus and Lower Esophageal Sphincter" In: R.K. Mittal: "Motor Function of the Pharynx, Esophagus, and its Sphincters", 2011, Morgan & Claypool Publishers, San Rafael (CA), USA
- Figure A. Illustration external and internal anal sphincter

## Description

### A. Field of the Invention

The present invention relates to methods of preventing or treating anal incontinence. In particular the present invention relates to the prevention or treatment of anal incontinence by injecting myoblasts into the external anal sphincter.

### B. Background of the Invention

The ability to maintain continence is fundamental for our well-being as social beings. The loss of anal continence results in physical, physiological and social handicaps. Generally it is thought, that primarily elderly and handicapped people suffer from anal incontinence, however, these symptoms can occur in people of every age. The spectrum of anal incontinence, *i.e.,* the loss of control of the content of the intestine, ranges from minor faeces marks in the underwear to the loss of flatus up to massive episodes of uncontrolled defecation of soft or solid faeces. The reasons for this can be multilayered and complex. Independently of the extremely impaired life quality for the affected individual, impaired anal continence results in a not to be underestimated cost factor for the public health system. In the USA more than 400 million dollars are spent per year for anal incontinence help programmes. Furthermore, anal incontinence is the second most frequent reason for hospitalisation in nursery homes (more frequently than dementia). One third of the elderly people in nursery homes or hospitals are incontinent for faeces.

Anal continence apparati were studied in more detail in the last decade due to the possibility of endoanal imaging techniques. This also let to an improved understanding of the anal continence mechanism and the factors responsible for maintaining anal continence. Anal continence requires the coordination of different and anatomic and physiological functions. An intact sensibility ensures the perception of the status of rectal filling and recognition of the quality of faeces. A functional motoric innervation enables the sphincter to respond to an increased anal "closing request" in an appropriate manner. Finally, an anatomically intact sphincter apparatus provides for the occlusion of the anal canal. The dysfunction of one of these functions results in a disturbance of anal continence.

Two skeletal muscles are important for the voluntary control of the continence organs: the *Musculus sphincter ani externus,* and the *Musculus puborectalis* as a part of the *Musculus levator ani.* It is likely that the remaining *Diaphragma pelvis* (*M. pubococcygeus, M. ischiococcygeus, M. iliococcygeus*) plays a more or less supportive role. The external anal sphincter is supported by the *N. pudendus.* Both muscles, *Musculus sphincter ani externus* and the *Musculus puborectalis,* can maintain a constant tonus directly proportional to the volume/amount of rectal filling, which tonus decreases with start of the defecation process. The constant base line tonus of the *M. puborectalis* results in a "contortion" of the ano rectal transition towards the symphysis forming an angle of 90° between the anal canal and the rectum. This anorectal angle also contributes to the maintenance of anal continence. A further function of the *M. puborectalis* is to retain at least partially formed faeces, if the external anal sphincter has been damaged.

Control over flatus or liquid faeces is not possible by *M. puborectalis.* The anal continence for these faeces types is provided by an interaction of the internal and external anal sphincters. The haemorrhoidal cushions provide for air tight occlusion. In the resting state the anal canal is occluded by the constant tonic activity of the external anal sphincters and the base line resting pressure of the internal anal sphincter. The internal anal sphincter, which is a continuation and enlargement of the circular smooth muscle layer of the colon, provides for about 75-85% of the base line pressure of the closed anal canal. The activity of this smooth muscle component is completely inhibited by a rectal distension, the so called rectal anal inhibitory reflex. This relaxation is accompanied by a reflex contraction of the external anal sphincter and *M. puborectalis* to prevent defecation.

For the discrimination of the different faeces qualities - solid faeces, liquid faeces, flatus, *etc.* - the anal sensibility plays a crucial role. Due to its extensive sensoric innervation the anal canal is suited for this objective very well. For anal continence, with regard to sensory aspects, in principle two main components are responsible: On the one hand the rectum recognises the level of filling of the ampulla recti, on the other hand the upper part of the anal canal is capable of determining the type of the intestinal content. The sensory feedback of these two sensors is essential for the coordinated activity of the sphincter musculature. This provides for the astonishing ability to let flatus pass while simultaneously the continence for liquid and solid faeces is maintained.

In the context of vaginal births about 0.5 to 2% of all women suffer from a perineal rupture of the third or fourth grade (rupture of the anal mucosa membrane). Currently, several studies try to discover the risk factors for the occurrence of high-grade perineal ruptures. Forceps delivery, primiparity, a weight of the child over 4 kg, as well as position anomalies of the child are considered to be predisposing factors resulting in a significantly increased frequency of an injury of the perineum. In most cases, the first aid applied in cases of perineal ruptures is inadequate. In one study covering 38 women, which had been surgically treated after a perineal rupture of the third grade, 14 women became incontinent for faeces three months later: nine for solid or soft faeces, five for flatus. Another study compared women with a perineal rupture of the third grade with women without perineal rupture. It was shown that half of the women with perineal rupture suffered from anal incontinence or an imperative defecation urgency. By using sonographical means it was shown, that in 85% of the cases an anal sphincter defect could be detected in these women, while such a defect was only detected in 33% of the women without obvious perineal rupture.

The identification of the reasons for these poor functional results requires further studies. It is possible that a tissue edema and the dilatation of the tissue immediately post-partal hamper an exact anatomical reconstruction. However, in most cases severe injuries are either not identified or are surgically treated in an inadequate manner. Predictive for the persistence of anal incontinence is a transient anal incontinence after a perineal rupture of grade 3 or 4.

In a further study, women with a persisting anal sphincter defect after a perineal rupture of grade 3 or 4 were subjected to anterior overlap repair. Prior to the surgical treatment the women were incontinent for solid as well as for soft faeces. 80% of the women treated in this manner regained complete anal continence; only a few remained incontinent for flatus. However, these promising results were rectified in long-term studies. Five years later, only about 50% of the relevant patients reported a "relative" improvement of their anal continence status. Physiological studies verified theses results. It looks like, as if the traumatically lesions, as a result of giving birth, also affect the motoric innervation.

Studies with women, having a perineal rupture of grade 3 or 4 and exhibiting the symptoms of anal incontinence, show that a significant aggravation of the symptoms occurred after a further vaginal birth. Even those women who initially remained without symptoms after perineal rupture, suffered after a further vaginal delivery much more frequently from symptoms of anal incontinence.

As mentioned above, current treatment methods primarily aim at the surgical correction of the tearing of the sphincter. This leads to short-term improvement of the symptoms as mentioned above.

Mild forms of anal incontinence can be treated with conservative methods of treatment, which can result in an improvement of the symptoms. However, in more serious cases, the respective surgical intervention results often in a short-term improvement with a small chance of success.

Conservative methods of treatment comprise a dietary change in addition to an increased uptake of fibres as well as in cases with impaired anal sensitivity, the usage of anal tampons and rectal enemas. The intake of loperamid, if necessary, also in combination with bile acid binding substances, reduces the intestinal motility and increases the pressure of the anal sphincter muscle (Hallgren *et al.,* 1994). A new form of therapy utilizes locally applied locally estrogen for postmenopausal women, however, randomised studies are lacking (Donnelly *et al.,* 1997).

A biofeedback therapy is for the patients simple, cheap and without side effects. The aim of such trainings is an increase of the tonus of the anal sphincter as reaction on an increase of the rectal filling. Three approaches are applied: 1. A coordinated training, in which the patient learns to react on an increased rectal filling by a contraction of the sphincter. 2. Training of the sensoric mechanisms in order to recognise smaller rectal filling amounts. 3. The isolated contraction of the anal sphincter and reduction of states of panic. The prospects of success for this method are in between 38 % and 100 %, wherein the interpretation of "success" varies significantly.

However, a distinct number of patients require a surgical intervention: Most frequently, sphincter repair (approximal or overlapping) is applied for the acute treatment of traumatically injuries after giving birth, but also secondarily after injuries of the anal sphincter caused by other circumstances. The short-term prospects are good, long-term results are poor (Malouf *et al.,* 2000).

A non-stimulated muscle transponate (*M. gracilis* or *M. gluteus*) shows good results in children. However, the improved status deteriorates over the years. Furthermore, the data documentation of this surgery method is insufficient. The method was all but completely replaced by the introduction of stimulated muscle transponates.

An electrically stimulated neo-sphincter by using the *M. gracilis,* can be applied to patients, where conventional methods failed. The rate of complication is, however, very high, indicating that the surgery should be conducted in centres, which have a great experience with this technique.

The artificial anal sphincter, a sphincter system made of silicon, whose sleeve is convoluted around the anal canal and filled to provide for the closing of the anal canal, is only applied to patients, which have aside of a very heavy form of anal incontinence also the physical and physiological prerequisites to handle such a system correctly. The morbidity rate after such an implantation is very high and in about one third of the patients an explantation is necessary. This surgery is also reserved to centres, which have highly specialised on this method.

Stimulation of the sacral nerve is a promising technique, but only if applied to a highly selected, very small group of patients. In this approach a thin electrode is implanted into the foramen 3 of the os sacrum. The anal sphincter should be intact. Long-term results are not yet available.

Only a very small amount of data is available for the topic "injectable biomaterials and faecal incontinence." Additionally, standardised operating procedures are lacking for this approach as well as results covering longer observation periods (Vaizey *et al.,* 2005). Some examples for such material are:
- Polyref: a mixture of polytetrafluoroethylene, glycerol and polysorbit; induces foreign body granulomas.
- PTQ implants: Formally called macroplastique; was applied under the name of bioplastic in anal incontinence cases in the years 2001 and 2002. The high viscosity of the product complicated a precise injection. So far only treatment of a total of 16 patients with faeces incontinence is published, no long-term observation data are available (Malouf *et al.,* 2001; Kenefick *et al.,* 2002). There is a risk of formation of foreign body granulomas.
For stress urinary incontinence it has been proposed to inject muscle-derived cells into the injured site in order to ameliorate stress urinary incontinence. However, stress urinary incontinence is not comparable to anal incontinence since the causes for the two different diseases are completely different. In addition, the two systems (urinary vs. anal) also do not have similar function. The urinary tract has to provide sufficient control of liquids only. The rectum is capable of controlling solid, fluid as well as gaseous contents. This necessitates completely different sensory requirements. Therefore, the anatomy is quite different between the genitourinary tract and the rectum. For example, while there is an external anal sphincter muscle encircling the rectum, there is no equivalent encircling completely the urethra. Furthermore, dorsal of the urethra hardly any striated muscles can be found in the adult male, while the external anal sphincter of the rectum is a striated muscle.

WO 96/40304 discloses the use of muscle-derived cells together with injectable biocompatible polymers for use in treating incontinence.

US 2003/077244 discloses polyacrylamide hydrogels as endoprosthetic devices for bulking the urethra, rectum or colon, or urether in order to increase resistance in these conduits for treatment of urinary incontinence, anal incontinence, and vesicouretal reflux.

US 2006/257445 discloses a suspension of smooth muscle cells in a biodegradable non-proteinaceous polymer solution that forms an ionically cross linked hydrogel having the cells dispersed therein when injected in vivo into the wall of a lumen in the vicinity of or around the vicinity of a sphincter to treat.

However, none of the afore-mentioned applications discloses the use of myoblasts for the prevention and treatment of anal incontinence.

US 2007/224173 discloses a system and method for obtaining a solution of heterologous cells for introduction to the body.

WO 2008/031957 discloses a method of therapy comprising the functional treatment of sphincters with cells selected from skeletal muscle stem cells, muscle stem cells, muscle precursor stem cells, myoblasts or satellite cells, wherein the therapy comprises the functional treatment of anal incontinence. The application does not disclose the application of cells into the external anal sphincter.

WO 2008/076435 discloses the use of muscle-derived progenitor cells (MDC) that show long-term survival following introduction into soft tissues, methods of isolating MDCs, and methods of using MDC-containing compositions for the augmentation of human or animal soft tissues. The application of the cells is conducted into the anal sphincter.

In view of the above-mentioned limitations and complications of treating anal incontinence, new and effective modalities in this area are needed in the art.

### SUMMARY OF THE INVENTION

In accordance with the present invention, muscle cell injection therapy using muscle-derived cells is provided as an improved and novel means for preventing and treating anal incontinence. As but one advantage, muscle-derived cell injection can be autologous, so that there will minimal or no allergic reactions. Myogenic cells such as myoblasts are not absorbed; thus, they can provide a better improvement and cure rate.

Myoblasts, the precursors of muscle fibers, are mononucleated muscle cells which differ in many ways from other types of cells. Myoblasts naturally fuse to form post-mitotic multinucleated myotubes which result in the long-term expression and delivery of bioactive proteins. Myoblasts have been used for gene delivery to muscle for muscle-related diseases, such as Duchenne muscular dystrophy, as well as for non-muscle-related diseases, e.g., gene delivery of human adenosine deaminase for the adenosine deaminase deficiency syndrome; gene transfer of human proinsulin for diabetes mellitus; gene transfer for expression of tyrosine hydroxylase for Parkinson's disease; transfer and expression of Factor IX for hemophilia B, delivery of human growth hormone for growth retardation.

The use of myoblasts to treat muscle degeneration, to repair tissue damage or treat disease is disclosed in U.S. Patents 5,130,141 and 5,538,722. Also, myoblast transplantation has been employed for the repair of myocardial dysfunction (Robinson *et al.,* 1995; Murry *et al.,* 1996; Gojo *et al.,* 1996; Zibaitis *et al.,* 1994).

The present invention provides a muscle-derived cell for use in preventing and treating of anal incontinence in a subject, wherein said cells are myoblast cells, wherein said cells are autologous or heterologous to said subject, and wherein the muscle-derived cell is to be administered in the external anal sphincter.

Described are new and effective methods for the prevention or treatment of anal incontinence, by delivering muscle-derived cells to muscle tissues of the rectum, to the anal sphincter system, and to the external anal sphincter. Therefore, described are methods of preventing or treating anal incontinence, wherein the method comprises the following steps: (a) introducing of an injection device through the skin of a patient, (b) moving the injection device forward until the injection device reaches the injection site of interest, and (c) injecting of previously obtained muscle-derived cells via said injection device into said injection site of interest, wherein the injection site of interest is, or is adjacent to, muscle-tissue providing for anal continence. Step (c) may further comprise withdrawing the injection device from the site of interest while, at the same time, said muscle-derived cells are dispensed from said injection device along a least a portion of the injection canal created by the moving of said injection device into the injection site of interest, thereby creating an injection band. The injection band may be no more than about 600 µm in diameter and/or the length of the injection band may be as long as the muscle being injected. The muscle-tissue providing for anal continence is the external anal sphincter. Also described is that the muscle-tissue for anal continence is *M. puborectalis.*

Additionally, described are methods of preventing or treating anal incontinence, wherein the method comprises the following steps: (a) introduction of an injection device into the rectum of a patient, (b) moving the injection device forward along the rectum until the injection device reaches the plane of the injection site of interest; (c) penetrating the rectum wall with the injection device; and (d) moving the injection device forward until the injection device reaches the injection site of interest, and subsequently, (e) injecting of previously obtained muscle-derived cells via the injection device into the injection site of interest, wherein the injection site of interest is, or is adjacent to, muscle-tissue providing for anal continence. Step (e) may further comprise withdrawing the injection device from the site of interest while, at the same time, said muscle-derived cells are dispensed from said injection device along a least a portion of the injection canal created by the moving of said injection device into the injection site of interest, thereby creating an injection band. In one embodiment the injection band may be no more than about 600 µm in diameter and/or the length of the injection band may be as long as the muscle being injected.

In one embodiment the muscle-derived cells to be injected can be autologous myoblasts. When practicing the present invention these cell types may be injected into or adjacent to an injured muscle tissue providing for anal continence, i..e. an injured anal sphincter externus as means of prevention or treatment for anal incontinence.

The previously obtained muscle-derived cells, *i.e.,* obtained prior to practicing the methods of the present invention, can be cultured cells which can generate sufficient quantities of muscle cells for repeated injections. Alternatively, the muscle-derived cells are primary cells.

Therefore, the present invention also provides a simple prophylaxis approach or treatment method for women and men with anal incontinence or in risk of developing anal incontinence by using autologous myoblasts to enhance their anal sphincters. Such muscle-derived cell therapy allows repair and improvement of damaged anal sphincter. In accordance with the present invention the treatment comprises a needle aspiration to obtain myoblasts, for example, and a brief follow-up treatment to inject cultured and prepared cells into the patient. Also according to the present invention, autologous muscle cell injections using myoblasts harvested from and cultured for a specific anal incontinence patient can be employed as a non-allergenic agent to bulk up the rectum wall, thereby enhancing coaptation and improving the anal sphincter muscle. In this aspect of the invention, simple autologous muscle cell transplantation is performed, as discussed above.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more," "at least one," and "one or more than one."

The term "about" means that the value stated, plus or minus 5% of the stated value, or the standard error for measurements of the given value, are contemplated. The invention will be better appreciated and understood when considered in conjunction with the following description and the accompanying drawing.

### BRIEF DESCRIPTION OF THE DRAWINGS

The following drawing form part of the present specification and are included to further demonstrate certain aspects of the present invention. The invention may be better understood by reference to the drawing in combination with the detailed description of specific embodiments presented herein.

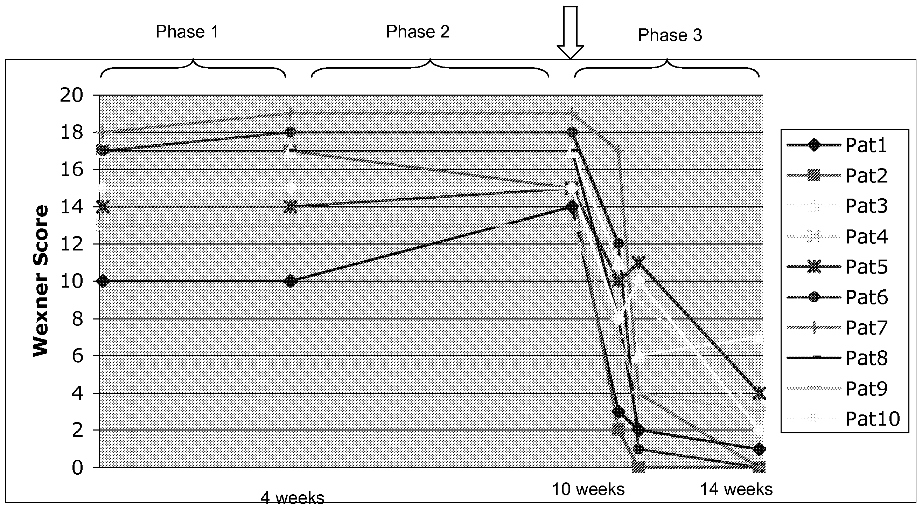

**FIG. 1** illustrates the "Wexner score" in 10 human patients before and after the implantation of autologous myoblasts into the external anal sphincter. In "Phase 1" (0-4 weeks) all patients had to perform pelvic floor training and electro-stimulation in the same way as after the cell injection. Only after non significant reduction of the "Wexner score" the muscle biopsy was taken. In the time of cell culture (approx. 6 weeks) the patients was introduced to go on with pelvic floor training (Phase 2). After the implantation of the expanded myoblasts (red arrow) the patients repeated the training program from Phase 1. A significant reduction of the "Wexner Score" can be seen after the cell injection in contrast to electro stimulation alone.

### DETAILED DESCRIPTION OF THE INVENTION

### A. Definitions

The term "anal incontinence," as used herein, refers to any undesired loss of intestine content through the anus, like flatus, liquid or solid faeces. The term comprises all three severity grades: Grade 1 = only gaseous, grade 2 = liquid and soft feces, grade 3 = solid, formed feces.

The term "anal sphincter" or "anal sphincter apparatus," as used herein, refers in particular to the *Musculus sphincter ani externus* and the *Musculus puborectalis* as a part of the *Musculus levator ani.* However it also includes *M. pubococcygeus, M. ischiococcygeus, M. iliococcygeus* and *N. pudendus.*

The term "muscle derived cell" refers to myoblasts, which can be primary cells and/or in vitro cultured cells and alternatively to other cells with myogenic potential (*e.g.*, from liposuctioned tissue or other stem cell harbouring tissues such as bone marrow). The term also comprises cells derived from adipose which can be isolated and used for culturing of skeletal muscle cells.

The term "penetration," as used herein, refers to a process of introducing an injection device, for instance a needle into a body tissue without effecting the injection process yet.

The term "injection," as used herein, refers to the expulsion of an injection solution comprising above mentioned cells out of an injection device into a specific site within the human body, in particular into or adjacent to muscle-tissue providing for anal continence. The injection process can be, but is not limited to, static, *i.e.,* the injection device remains at the position reached. Alternatively, the injection process is dynamic. For instance, in some embodiments of the present invention the injection occurs simultaneously with the retraction of the injection device from the site of injection.

The term "injection site," as used herein, refers to a site within the human body, such as close to or being muscle-tissue providing for anal continence, at which the injection process is initiated. The injection site needs not to be identical with the site where the injection process ends.

The term "injection device," as used herein, refers to any device suitable for penetrating human tissue in order to reach an injection site of interest and capable of delivering solutions, in particular solutions comprising muscle-derived cells to the injection site of interest.

The term "faeces incontinence," as used herein, refers only to the undesired loss of liquid or formed faeces through the anus.

The term "passive incontinence," as used herein, refers to a lack of sensory recognition of loss of faeces. This comprises low anal base line pressure values and a lacking sensoric ability of the anal and rectal mucosa.

"Imperative defecation" or "imperative urgency," as used herein, refers to the lacking ability of a person to delay defecation for more than five minutes. Such a patient has go to the toilette immediately.

### B. Cells and Cell Types for use in the Present Invention

A number of muscle-derived and/or myogenic cells are suitable for use with the methods of the present invention. Non-limiting examples of such cells include myoblasts, fibroblasts, and muscle-derived stem cells which reside in muscle tissue. Cells with myogenic potential (*e.g.*, from liposuctioned tissue or other stem cell harbouring tissues (bone marrow), or adipose derived cells), could be used.

In the present invention, myoblasts are used in the repair of the anal sphincter apparatus. In particular, the cells for use in the present invention have to be capable to fuse (syncytium of at least three cells) and to establish an oriented, contractile cytoskeleton (actin-myosin sequence).

In accordance with the present invention, myoblasts, may be primary cells or cultured cells. They may be histocompatible (autologous) or nonhistocompatible (allogeneic) to the recipient, including humans. Particular embodiments of in the present invention are myoblasts and muscle-derived cells, including autologous myoblasts and muscle-derived cells which will not be recognized as foreign to the recipient. In this regard, the myoblasts can be matched *vis-á-vis* the major histocompatibility locus (MHC or HLA in humans). Such MHC or HLA matched cells may be autologous. Alternatively, the cells may be from a person having the same or a similar MHC or HLA antigen profile. The patient may also be tolerized to the allogeneic MHC antigens. The present invention also encompasses the use of cells lacking MHC Class I and/or II antigens, such as described in U.S. Patent 5,538,722.

Establishment of a primary muscle-derived cell culture from isolated cells of muscle tissue can be obtained by methods well known to a person skilled in the art, *e.g.,* via a muscle biopsy. Such muscle biopsy serving as the source of muscle-derived cells can be obtained from the muscle at the site of injury or from another area that may be more easily accessible to the clinical surgeon. As mentioned above, the muscle-derived cells need not necessarily to be obtained from the patient to be treated. However, an embodiment of the invention is where the muscle biopsy is taken from the patient suffering from anal incontinence. The site of the biopsy is not restricted but may be a skeletal muscle, such as from the upper arm. The size of the biopsy may comprise approximately 1 cm x 1 cm x 1 cm or bigger. From this biopsy sample satellite cells, *i.e.,* cells capable to fuse (syncytium of at least three cells) and to establish an oriented, contractile cytoskeleton (actin-myosin sequence) are isolated and cultured. About 60 to about 500 million cells may be cultured for a single treatment. Additionally, a blood sample can be obtained from the patient, which is subsequently used for cultivation of the cells *in vitro.* Alternatively, fetal bovine serum is used for cultivation. Myoblasts in cell culture can be further purified using an established technology (Rando and Blau, 1994) or other methods. These muscle cells are cultivated *in vitro.*

In a muscle biopsy, a small area of muscle tissue generally contains enough myogenic cells to produce millions of muscle-derived cells in culture. For myoblasts, once the cells are isolated and grown in culture, it is easy to distinguish pure myoblasts from other cell types, since myoblasts fuse to form elongated myotubes in vitro. In addition, desmin, a myogenic specific marker protein, can be used to determine the myogenicity index of the cell culture without the requirement of differentiation.

### C. Injection Procedures

In general, injecting muscle-derived cells, including myoblasts, muscle-derived stem cells or other cells with myogenic potential (see above), into a given tissue or site of injury comprises a therapeutically effective amount of cells in solution or suspension, e.g., about 1 x 10⁶ to about 6 x 10⁶ cells per 100 µl of injection solution. The injection solution is a physiologically acceptable medium, with or without autologous serum. Physiological acceptable medium can be by way of non-limiting example physiological saline or a phosphate buffered solution.

In one embodiment of the present invention, muscle-derived cell injection comprises autologous myoblast injection, into the external anal sphincter is employed as a treatment for anal incontinence to enhance, improve, and/or repair the external anal sphincter. The myoblasts, are injected into the external anal sphincter and survive and differentiate into myofibers to improve sphincter function. The feasibility and survival of myoblast injection into the external anal sphincter has been verified. In accordance with this embodiment, autologous myoblasts cell injections (*i.e.,* muscle-derived cells harvested from and cultured for a specific anal incontinence patient) can be used as a nonallergenic agent to bulk up the rectum wall, thereby enhancing coaptation and improving the anal sphincter muscle function by integration into the striated muscle fibres of the muscle. For this purpose also fibroblasts can be used which are capable of building up an extracellular matrix consiting of collagen, which provides for the bulking effect.

In accordance with the present invention, autologous myoblast cells administered directly into the external anal sphincter exhibit long-term survival. Thus, autologous myoblast injection results in safe and nonimmunogenic long-term survival of myofibers in the anal sphincter.

In a particular embodiment according to the invention, about 50 to about 200 µl of a muscle-derived cell suspension comprising myoblasts (with a concentration of about 1 x 10⁶ to about 6 x 10⁶ cells per 100 µl of injection solution) are injected into the external anal sphincter. The injection device can be connected to a container containing the cell suspension to be injected.

In another embodiment, the injection step may comprise several individual injections, such as about 20 to about 40 injections of muscle-derived cell suspension, wherein in each injection about 50 to about 200 µl of a muscle-derived cell suspension are injected and wherein each injection is applied to another region of the anal sphincter. However, these parameters have to be considered as being merely exemplarily and the skilled artisan will readily be able to adapt these procedures to the treatment requirements for each individual patient.

In another embodiment of the present invention, the movement of the injection device towards the external anal sphincter is monitored by sonography and/or EMG (electromyography) means. In a particular embodiment, a transrectal probe is introduced and the position of the transrectal probe is adjusted optimally for the treatment of the external anal sphincter with the methods according to the invention. In another particular embodiment, the muscle-derived cells are implanted in the area surrounding the external anal sphincter defect and/or especially in the area of the external anal sphincter defect. The patient can start the next day after injection of cells with physical exercises to further the treatment of anal incontinence according to the invention.

In another embodiment, the treatment is repeated. The treatment can be repeated *e.g.* within one year after the last treatment, after 10, 9, 8, 7, 6, 5, 4, 3, 2 or 1 month(s) after the last treatment or within 1 to 8 weeks, preferably 2 to 3 weeks, or 10 to 20 days after the last treatment. In particular, the treatment can be repeated within 2 to 3 weeks after the last treatment with cells from the very same cell culture as used for the prior treatment. This approach allows for a reduced injection volume per injection and gives the cells more time to adapt and to integrate and to build up the muscle. In an even more specific embodiment, the injections are repeated in time intervals of 2 to 3 weeks until an improvement of anal continence is achieved.

In particular, the present invention comprises the injection of the myoblast cells into the external anal sphincter, such as into or adjacent to the site of injury. The method of the present invention is particularly useful for treatment of anal incontinence if the *M. puborectalis* muscle is still intact but the external anal sphincter is injured.

As mentioned above, a particular penetration route is through the skin of a patient in parallel to the course of the rectum. However, it is also contemplated, that the penetration can occur directly from the rectum in the vicinity of the injured muscle. In particular, the penetration and injection process is monitored via sonographic imaging means. Additionally, an alternative penetration route is contemplated for women, that is, trans-vaginal injection. In this scenario, the injection device penetrates the wall of the vagina and is moved forward until it reaches the desired injection site. In particular, the penetration and injection process is monitored by sonographic and/or EMG (electromyography) imaging means in this scenario as well.

In another embodiment, the injection comprises injecting the muscle-derived cells in form of an "injection band." "Injection band," as used herein, refers to disposition of cells along the length, or a portion of the length, of the injection track, *i.e.,* along the canal created by insertion of the needle into the muscle tissue. In other words, following injection, the needle is withdrawn while, at the same time, cells are expelled from the syringe in a continuous or intermittent fashion with the injection needle is moved, in particular, retracted along the injection track. Such steady dispensing of cells provides for a continuous delivery of the injection solution, including cells, along the injection canal that is formed when the injection device/needle enters the target muscle-tissue. In a particular embodiment, the injection band or canal should have a diameter not bigger than about 600 µm, since this would lead to necrosis of the myoblasts cells in the center of the injection canal, and consequently, result in detrimental inflammation and other processes.

### D. Injection Devices and Sonographic Imaging

The injection device for use with the methods of the present invention may be any device capable of penetrating human tissue and capable of delivering solutions, in particular solutions comprising muscle-derived cells to a desired location within the organism of a subject, in particular of a human subject. The injection device can comprise, for instance, a hollow needle. The injection device may also be any type of syringe suitable for injecting muscle-derived cells. In more sophisticated embodiments, the injection device can be for example a injection gun, injecting the cell suspension by applying air pressure. In particular, the injection device is suited for keyhole applications and keyhole surgery, respectively.

By choosing an injection needle having a particular diameter, the injection volume per mm³ can be exactly pre-determined. The diameter of the injection needle will normally not exceed 5 mm, as this can lead to damage of the muscle structures.

Sonographic imaging means for monitoring the position and action of the injection device can be achieved by any standard ultrasonic imaging device known in the art. In addition to mono- or biplanar standard ultrasonic probes, also new ultrasonic technologies can be used, such as, for example, 3D-sonography or color Doppler sonography, *etc.* In a particular embodiment, as discussed above, the injection device comprises a sonographic imaging means.

Sonographic imaging can be performed in radial mode or in longitudinal mode. The radial mode is often used when introducing the injection device into the rectum of a patient as this provides for exact positioning of the tip of the injection needle at the desired injection site. The longitudinal mode may be used if the injection process of the muscle-derived cells into or adjacent to the damaged tissue is to be monitored. In addition, the modes can also be combined, *i.e.,* first monitoring the penetration process in a first mode until the site of injection is reached, second monitoring in a second mode the injection process, when the injection needle is retracted from the injection site. However, it has to be noted, that the methods of the present invention are not dependent on ultrasonic imaging.

### E. Forms of Anal Incontinence to be Treated with the Present Invention

In principle, any type of anal incontinence can be treated, as the strengthening of the anal muscle systems provides for a better control of the rectal filling. However, anal incontinence that results from perineal rupture, especially if the anal sphincter system and/or if the M. puborectalis is damaged and injured, is treated. Such perineal rupture can result from a broad variety of causes as outlined above. However, the cause of such perineal rupture is not in limiting for the application of the methods of the present invention. Patients may be treated with the methods of the present invention if they suffer from a perineal rupture of the third or fourth grade. This applies in particular to women which suffer from such perineal rupture after forceps delivery, primiparity, a weight of the child over 4 kg, or as a consequence of position anomalies of the child before birth. The methods of the present invention can also be applied after injury of the anal sphincter system and/or M. puborectalis due to surgical procedures. Additionally, the methods of the present invention can also be applied if there is only transient incontinence. Such treatment prevents development of full anal incontinence. Further anal incontinence disease states to be treated with the methods of the present invention are passive incontinence, faeces incontinence and imperative defecation.

It should be understood, and has already been indicated above, that the methods according to the present invention cannot only be used to treat patients already suffering from anal incontinence, *i.e.,* showing symptoms of anal incontinence, but may be applied to subjects not yet suffering from anal incontinence, but with increased risk of doing so, for example, in cases where the rectal musculature suffered damage from surgery, birth, accidents and so forth. Another example would be cases where the rectal musculature became thinner than in an healthy individual or where it degenerated due to other reasons. The methods of the present invention can provide a suitable prophylaxis to prevent onset of anal incontinence.

### F. References

U.S. Patent 5,130,141
U.S. Patent 5,538,722
Donnelly et al., Br. J. Obstet. Gynaecol., 104(3):311-315, 1997.
Gojo et al., Cell Transplantation, 5:581-584, 1996.
Hallgren et al., Dig. Dis. Sci., 39(12):2612-2618,1994.
Kenefick et al., Gut, 51(2):225-228, 2002.
Malouf et al., Dis. Colon Rectum, 44(4):595-600, 2001.
Malouf et al., Lancet, 355(9200):260-265, 2000.
Murry et al., J. Clin. Invest., 98:2512-2523, 1996.
Rando and Blau, J. Cell. Biol., 125:1275-1287, 1994.
Robinson et al., Cell Transplantation, 5:77-91. 1995.
Vaizey and Kamm, Br. J. Surg., 92(5):521-527, 2005.
Zibaitis et al., Transplantation Proceed., 26:3294, 1994.

## Claims

1. A muscle-derived cell for use in preventing and treating of anal incontinence in a subject, wherein said cells are myoblast cells, wherein said cells are autologous or heterologous to said subject, and wherein the muscle-derived cell is to be administered in the external anal sphincter.

2. The muscle-derived cell for use according to claim 1, wherein the myoblasts have been obtained from a donor and/or wherein the muscle-derived cell is a primary cell and/or wherein the administration of said cell is repeated.

3. The muscle-derived cell for use according to claim 1 and 2, wherein said cells are cultured prior to administration.

4. The muscle-derived cell for use according to claims 1 to 3 to be administered to the anal sphincter externus of a a subject, wherein said subject has suffered a rectal injury.

## Patentansprüche

1. Eine vom Muskel-abstammende Zelle zur Verwendung zum Vorbeugen und Behandeln von analer Inkontinenz in einem Subjekt, wobei die Zellen Myoblastenzellen sind, wobei die Zellen autolog oder heterolog zu dem Subjekt sind, und wobei die vom Muskel-abstammende Zelle in den externen analen Sphincter verabreicht wird.

2. Die vom Muskel-abstammende Zelle nach Anspruch 1, wobei die Myoblasten von einem Spender erhalten wurden, und/oder wobei die vom Muskel-abstammende Zelle eine primäre Zelle ist, und/oder wobei das Verabreichen der Zelle wiederholt wird.

3. Die vom Muskel-abstammende Zelle zur Verwendung nach Anspruch 1 und 2, wobei die Zellen vor dem Verabreichen kultiviert werden.

4. Die vom Muskel-abstammende Zelle zur Verwendung nach den Ansprüchen 1 bis 3, um in den analen Sphincter externus eines Subjekts verabreicht zu werden, wobei das Subjekt an einer rektalen Verletzung gelitten hat.

## Revendications

1. Cellule dérivée du muscle pour une utilisation dans la prévention et le traitement de l'incontinence anale chez un sujet, dans laquelle lesdites cellules sont des cellules myoblastes, dans laquelle lesdites cellules sont autologues ou hétérologues audit sujet, et dans laquelle la cellule dérivée du muscle est à administrer dans le sphincter anal externe.

2. Cellule dérivée du muscle pour une utilisation selon la revendication 1, dans laquelle les myoblastes ont été obtenus depuis un donneur et/ou dans laquelle la cellule dérivée du muscle est une cellule primaire et/ou dans laquelle l'administration de ladite cellule est répétée.

3. Cellule dérivée du muscle pour une utilisation selon la revendication 1 et 2, dans laquelle lesdites cellules sont cultivées avant administration.

4. Cellule dérivée du muscle pour une utilisation selon les revendications 1 à 3 est à administrer dans le sphincter anal externe d'un sujet, dans laquelle ledit sujet a souffert d'une lésion rectale.
